# EUROPEAN PATENT SPECIFICATION

(11) **EP 2 398 914 B1**
(45) Date of publication and mention of the grant of the patent: **30.09.2015**
(21) Application number: 10705853.9
(22) Date of filing: 19.02.2010
(51) Int. Cl.: C12Q 1/68

(54) **METHOD OF CELL-LINE IDENTIFICATION**
ZELLLINIENIDENTIFIZIERUNGSVERFAHREN
PROCÉDÉ D'IDENTIFICATION D'UNE LIGNÉE CELLULAIRE

(30) Priority: 23.02.2009 GB 0903026
(43) Date of publication of application: 28.12.2011
(73) Proprietor: GlaxoSmithKline Biologicals S.A., 1330 Rixensart (BE)
(72) Inventor: CASSART, Jean-Pol, B-1330 Rixensart (BE); LIENARD, Patricia, Anne-Laure, B-6041 Gosselies (BE)
(74) Representative: Robertson, James Stuart
(86) International application number: PCT/EP2010/052117
(87) International publication number: WO 2010/094763

(56) References cited:
- WO-A2-03/106706
- WO-A2-2006/094836
- US-A1- 2008 113 349
- CHALLIER C ET AL: "Differential expression of the ufo/axl oncogene in human leukemia-lymphoma cell lines" LEUKEMIA, MACMILLAN PRESS LTD, US, vol. 10, no. 5, 1 January 1996 (1996-01-01), pages 781-787, XP009132912 ISSN: 0887-6924
- TOUTENHOOFD SONJA L ET AL: "Characterization of the human CALM2 calmodulin gene and comparison of the transcriptional activity of CALM1, CALM2 and CALM3" CELL CALCIUM, vol. 23, no. 5, May 1998 (1998-05), pages 323-338, XP002580659 ISSN: 0143-4160
- MULE G ET AL: "A species-specific PCR assay based on the calmodulin partial gene for identification of Fusarium verticillioides, F. proliferatum and F. subglutinans" EUROPEAN JOURNAL OF PLANT PATHOLOGY, vol. 110, no. 5-6, June 2004 (2004-06), pages 495-502, XP002580660 ISSN: 0929-1873
- TAMEZ-GUERRA P ET AL: "Detection of genes encoding antimicrobial peptides in Mexican strains of Trichoplusia ni (Hubner) exposed to Bacillus thuringiensis" JOURNAL OF INVERTEBRATE PATHOLOGY, SAN DIEGO, CA, US LNKD- DOI:10.1016/J.JIP.2008.02.008, vol. 98, no. 2, 1 June 2008 (2008-06-01), pages 218-227, XP022684442 ISSN: 0022-2011 [retrieved on 2008-02-20]
- LIU MERRY ET AL: "Rapid identification and authentication of closely related animal cell culture by polymerase chain reaction" IN VITRO CELLULAR & DEVELOPMENTAL BIOLOGY ANIMAL, vol. 44, no. 7, August 2008 (2008-08), pages 224-227, XP002580661 ISSN: 1071-2690
- STACEY G N ET AL: "Authentication of animal cell cultures by direct visualization of repetitive DNA, aldolase gene PCR and isoenzyme analysis" BIOLOGICALS, vol. 25, no. 1, 1997, pages 75-85, XP002580662 ISSN: 1045-1056
- VIZIOLI J ET AL: "Antimicrobial peptides from animals: focus on invertebrates" TRENDS IN PHARMACOLOGICAL SCIENCES, ELSEVIER, HAYWARTH, GB LNKD- DOI:10.1016/S0165-6147(02)02105-3, vol. 23, no. 11, 1 November 2002 (2002-11-01), pages 494-496, XP004389613 ISSN: 0165-6147
- LOSI CLARETTA G ET AL: "An alternative method to isoenzyme profile for cell line identification and interspecies cross-contaminations: cytochrome b PCR-RLFP analysis" IN VITRO CELLULAR & DEVELOPMENTAL BIOLOGY ANIMAL, vol. 44, no. 8-9, October 2008 (2008-10), pages 321-329, XP002580663 ISSN: 1071-2690
- KLAUS G STEUBE ET AL: "Identification and verification of rodent cell lines by polymerase chain reaction" CYTOTECHNOLOGY, KLUWER ACADEMIC PUBLISHERS, DO, vol. 56, no. 1, 31 October 2007 (2007-10-31), pages 49-56, XP019571608 ISSN: 1573-0778

## Description

### FIELD OF THE INVENTION

The present invention relates to the field of the identification of cell-lines. More particularly it relates to identification of cell-lines through analysis by molecular techniques, and identifying variation in introns or determining whether a particular gene is present. This method is particularly useful for the quality control of cell-lines used for the production of pharmaceutical preparations.

### BACKGROUND OF THE INVENTION

Current methods performed in Quality Control (QC) use the isoenzyme technique. This method is based on the analysis of the electrophoretic mobilities of isoenzymes which differ between species. Mobility profiles can be established for each species using the combined analysis of the migration distances of enzymes such as: glucose-6-phosphate dehydrogenase (G6PD), aspartate aminotransferase (AST), lactate dehydrogenase (LDH) and malate dehydrogenase (MD). This method provides a means of determining the species of origin of a cell line and detecting cells cross-contaminated with cells from another cell line of different species (Stacey and Hockley, 2006). However, this test is labour intensive and time consuming. Liu *et al.* (2008) discloses identification and authentication of closely related animal cell culture by PCR amplification of the aldolase gene.

### SUMMARY OF THE INVENTION

The present inventors have demonstrated that cell-lines can be identified by a method more reliably and easily than current identification methodologies. The identification method of the present invention is based on the analysis of the size of intron sequences which differ between species. This method involves the amplification of the intron sequences by polymerase chain reaction (PCR). The present inventors have identified genes that are sufficiently conserved in the exon sequences to allow the annealing with the amplification PCR primers and sufficiently different in the intron sequence to allow discrimination based on the length polymorphism. Discrimination of these variations can thus be determined through analysis of these genomic regions.

The method allows identification of a number of cell-line types derived from vertebrates (e.g. MRC-5, Vero, CHO, FRHL-2, MDCK, Chicken Embryo Fibroblasts [CEF]), based on informative polymorphic introns found in two genes, calmodulin and Axl receptor tyrosine kinase. Further, using a gene specific to insect cell-lines, analysis to determine the presence or absence of an insect gene can help identify insect derived cell-lines, for example Hi-5.

Accordingly, the present invention provides a method of cell-line identification comprising one or more of the following steps:
(a) analysis of the calmodulin gene, wherein a fragment of the calmodulin gene is amplified by PCR and wherein the amplified fragment comprises the sequence between exon 3 and exon 4 of the calmodulin gene; and
(b) analysis of the Axl receptor tyrosine kinase, wherein a fragment of the Axl receptor tyrosine kinase gene is amplified by PCR, and wherein the amplified fragment comprises the sequence between exon 18 and exon 19 of the Axl receptor tyrosine kinase gene;
and wherein the cell-line is identified by the size of an intron in said genes.

### BRIEF DESCRIPTION OF FIGURES

**Figure 1** Analysis of the CALM3/4 PCR products.
**Figure 2** Analysis of the AXL 18/19 PCR products.
**Figure 3** Analysis of the PPATTA PCR product.

### DESCRIPTION OF THE INVENTION

The terms "comprising", "comprise" and "comprises" herein are intended by the inventors to be optionally substitutable with the terms "consisting of", "consist of", and "consists of", respectively, in every instance.

The present inventors have identified both conserved and variable regions in the genes encoding Calmodulin and Axl receptor tyrosine kinase. The variation in the genes allows for discrimination between the different cell-lines; these variations can be detected using any method that allows analysis of the nucleic acid. Furthermore, the conserved regions can be used to design complementary primers for either PCR amplification or sequencing reaction that can be used to analyse cell-lines from many sources. Insect-derived cell lines can be identified by detecting insect specific nucleotide sequences.

Accordingly, the present invention provides a method of cell-line identification as defined in the claims.

The present invention may be used to identify many types of cell-lines. The term "cell-line" as used herein refers to established cell-cultures grown *in vitro.* Cell-lines may be derived from any species of animal including mammals, birds and insects. In a particular embodiment the identification method of the present invention is used for cell lines used in the pharmaceutical industry. In a particular embodiment, the methods of the present invention are used to identify cell-lines used in the production of viruses that may be used in vaccines. The term "cell-line" encompasses continuous cell-cultures which are cell-cultures of morphologically uniform cells that can be propagated *in vitro* for an indefinite time. Accordingly, in one embodiment, methods of the invention can be used to identify and/or differentiate cell-lines selected from the group: MRC-5, Vero, Hi-5, CHO, FRHL2, and MDCK. The term "cell-line" also incorporates primary cell-lines, i.e. cells that are isolated directly from a subject. Accordingly, in one embodiment, the methods of the present invention are used to identify chicken derived cells, for example, CEF (chicken embryo fibroblasts).

The present inventors have identified genes calmodulin and Axl receptor tyrosine kinase which comprise both conserved exons and variable introns. Accordingly, in one aspect of the disclosure, there is provided a method of cell-line identification comprising the step: analysis of the calmodulin gene. In a further aspect there is provided a method of cell-line identification comprising the step: analysis of the Axl receptor tyrosine kinase gene.

An insect specific gene has also been identified, allowing discrimination between insect derived cells and cells derived from other organisms, for example mammalian or avian cells. Accordingly, in a further aspect of the disclosure there is provided a method of cell-line identification comprising the step: analysis of an attacin gene.

The term "analysis" refers to any analytical technique that will allow the skilled person to differentiate/discriminate nucleic sequences from different cell lines. There are a large number of techniques suitable for this purpose known to the skilled person, these techniques include but are not limited to: Polymerase Chain Reaction (PCR) amplification, sequencing, hybridisation, and restriction length fragment polymorphisms (RFLP). In one aspect of the disclosure there is provided a cell-line identification method of the invention wherein the analysis method is selected from the group consisting of Polymerase Chain Reaction (PCR) amplification, sequencing, hybridisation and restriction length fragment polymorphisms (RFLP).

The term "calmodulin gene" is well known to the person skilled in the art and as used herein is intended to mean a gene that encodes a polypeptide comprising at least one EF-hand motif that is capable of binding calcium. Calmodulin plays a major role in transmitting intracellular Ca²⁺ signals to a wide variety of effector systems (Rhyner et al., (1994) Euro J Biochem 225: 71-81). In mammals, 3 genes (CALM1, CALM2 and CALM3) encode the single, highly conserved, calmodulin (CaM) protein (Berchtold et al., (1993) Genomics 16: 461-465). In particular embodiments of the invention the term calmodulin gene is a nucleic acid sequence that encodes a polypeptide of SEQ ID NO: 7 or encodes an amino acid sequence having 70%, 75%, 80%, 85%, 90%, 95%, 96%, 97%, 98% or 99% sequence identity to SEQ ID NO:7.

The term "attacin gene" as used in herein refers to nucleic acid sequences that encode insect antibacterial peptides known as attacins and attacin-like proteins, including basic and acidic attacins (see for example Boman et al., (1991) Eur J. Biochem 201: 21-31). The term encompasses genomic sequences that encode attacins, proattacins and pre-proattacins; these terms are well known to those skilled in the art. Attacins are polypeptides that are specific to insects and are thus not found in mammals and or birds. In particular aspects of the disclosure the term attacin gene is a nucleic acid sequence that encodes a polypeptide of SEQ ID NO: 9 or polypeptides with amino acid sequences that have 70%, 75%, 80%, 85%, 90%, 95%, 96%, 97%, 98% or 99% sequence identity to SEQ ID NO: 9.

The term "Axl receptor tyrosine kinase" is well know to the person skilled in the art and is used herein to mean transmembrane proteins involved in intracellular signalling which is triggered through activation of the tyrosine kinase domain and the subsequent phosphorylation of a variety of substrates and which is a tyrosine kinase receptor part of the Axl family. Axl receptor tyrosine kinases are ubiquitously expressed in a number of cell lines including those of epithelial, mesenchymal and haematopoietic origin, as well as non-transformed cells (Hafizi & Dahlhbäck, 2006 [Cytokine & Growth Factor Reviews 17: 295-304]).

The transforming activity of the Axl receptor tyrosine kinase demonstrates that the receptor can drive cellular proliferation. Although the function of the Axl receptor tyrosine kinase in nontransformed cells and tissues was unknown, Varnum et al. (1995, Nature, 373: 623-626) suspected that it may involve the stimulation of cell proliferation in response to an appropriate signal, *i.e*., a ligand that activates the receptor. Varnum *et al.* (1995) purified an Axl receptor tyrosine kinase stimulatory factor and identified it as the product of the growth arrest-specific gene-6 (GAS6). The Axl tyrosine kinase gene encodes a tyrosine kinase receptor which regulates cell growth and differentiation (O'Bryan et al., (1991) Mol Cell Biol 11: 5016-31). The genomic organization of the human Axl tyrosine kinase gene locus was described by Schulz et al., (1993 Oncogene 8: 509-13). Human Axl tyrosine kinase gene contains 20 exons that are distributed over a region of 44 kb.

In particular embodiments of the invention the term Axl receptor tyrosine kinase gene means a nucleic acid sequence that encodes a polypeptide of SEQ ID NO: 8 or polypeptides with amino acid sequences that have 70%, 75%, 80%, 85%, 90%, 95%, 96%, 97%, 98% or 99% sequence identity to SEQ ID NO: 8.

In a further aspect, there is provided a method of cell-line identification comprising the following steps: (a) analysis of the calmodulin gene; and (b) analysis of the Axl receptor tyrosine kinase gene.

In a further aspect, there is provided a method of cell-line identification comprising the following steps: (a) analysis of the calmodulin gene; and (b) analysis of an attacin gene.

In one aspect, there is provided a method of cell-line identification comprising the folowing steps: (a) analysis of an attacin gene; and (b) analysis of the Axl receptor tyrosine kinase gene.

In a further aspect, there is provided a method of cell-line identification comprising the following steps: (a) analysis of the calmodulin gene; (b) analysis of the Axl receptor tyrosine kinase gene; and (c) analysis of an attacin gene.

It is envisaged that the skilled person may wish to use a variety of molecular techniques in order to identify one or more cell lines. For example, in certain aspects of the disclosure the skilled person may use one or more or any combination of the following techniques: PCR, sequencing, hybridisation and RFLP. Accordingly, the methods of the disclosure may be performed using one or more of the molecular techniques selected from the list comprising PCR, sequencing, hybridisation and RFLP, or any combination thereof.

In a particular aspect of the disclosure there is provided a method of cell-line identification comprising the steps comprising one or more of the following steps: (a) PCR amplification of a calmodulin gene or fragment thereof; (b) PCR amplification of a Axl receptor tyrosine kinase gene or fragment thereof; or (c) PCR amplification of an attacin gene or fragment thereof; or any combination thereof.

The term "fragment" as herein described refers to any nucleic acid sequence of a gene, that is not the whole gene sequence, comprising at least 50, 60, 70, 80, 90, 100, 110, 120, 130, 140, 150, 160, 170, 180, 190, 200, 400, 600, 800, 1000, 1200, 1400, 1600 or more nucleotides.

In a further aspect of the disclosure there is provided a method of cell-line identification comprising one or more of the following steps: (a) hybridisation of a probe to the calmodulin gene or fragment thereof; (b) hybridisation of a probe to an Axl receptor tyrosine kinase gene or fragment thereof; or (c) hybridisation of a probe to an attacin gene or fragment thereof; or any combination thereof.

In one aspect of the disclosure there is provided a method of cell-line identification comprising one or more of the following steps: (a) sequencing of the calmodulin gene or fragment thereof; (b) sequencing of an Axl receptor tyrosine kinase gene or fragment thereof; or (c) sequencing of an attacin gene or fragment thereof; or any combination thereof.

The genes encoding calmodulin comprise introns that vary in size between different cell-lines.

Accordingly, in an aspect, the cell-line is identified by the size (number of nucleotides) of the amplicon (the amplified product following PCR). The inventors have determined that introns within the calmodulin and Axl receptor tyrosine kinase genes vary in size between different species and/or strains. Accordingly, in a particular aspect of the disclosure cell-lines can be differentiated and/or determined by the size of a particular intron between any two exons. In a particular aspect, cell-lines are determined by the size of the amplicon derived from either the calmodulin gene and/or the Axl receptor tyrosine kinase gene. In one aspect there is provided a method of cell-line identification comprising the step: PCR amplification of the calmodulin gene or fragment thereof.

PCR amplification of a fragment of the calmodulin gene allows identification and/or differentiation of cell-lines as the same genes from different cell-lines differ in the length of introns. Thus primer sets that are designed to amplify one or more of the introns found in the calmodulin gene can be used to identify and/or differentiate cell-lines. Accordingly, in one aspect of the disclosure there is provided a method of cell-line identification comprising the step: PCR amplification of one or more of the calmodulin introns or a fragment thereof. In a further aspect, there is provided a method of cell-line identification comprising the step: PCR amplification of the intron between exons 3 and 4 of the calmodulin gene.

In one aspect there is provided a method wherein a fragment of the calmodulin gene is amplified by PCR and in a further aspect there is provided a method wherein the amplified fragment comprises the sequence between exon 3 and exon 4 of the calmodulin gene.

In a further aspect there is provided a method wherein a fragment of the calmodulin gene is amplified using at least one of the primers selected from SEQ ID NO: 1 and SEQ ID NO: 2 or functional derivative thereof.

The Axl receptor tyrosine kinase gene has conserved exons and introns that vary between different cell-lines. Accordingly, in one aspect there is provided a method of cell-line identification comprising the step: PCR amplification of an Axl receptor tyrosine kinase gene or fragment thereof. PCR amplification of a fragment of the Axl receptor tyrosine kinase gene allows differentiation between cell-lines as different cell-lines differ in the length of introns. Thus primer sets that are designed to amplify one or more of the introns found in Axl receptor tyrosine kinase genes can be used to differentiate between differing cell-lines; thus amplification of an intron that varies in length between different cell-lines may be used in the methods of the disclosure. Accordingly, in one aspect there is provided a method of cell-line identification comprising the step: PCR amplification of one or more Axl receptor tyrosine kinase gene introns or fragment thereof. In a further aspect, there is provided a method of cell-line identification comprising the step: PCR amplification of the intron between exons 18 and 19 of Axl receptor tyrosine kinase gene. In one aspect there is provided a method a fragment of the Axl receptor tyrosine kinase gene is amplified by PCR. In a further aspect there is provided a method wherein the amplified fragment comprises the sequence between exon 18 and exon 19 of the Axl receptor tyrosine kinase gene. Suitably, the method wherein a fragment of the Axl receptor tyrosine kinase gene is amplified uses at least one of the primers selected from SEQ ID NO: 3 and SEQ ID NO: 4 or functional derivative thereof.

The cell-type can be determined by the presence or absence of the gene *i.e*. the particular cell-line may be differentiated from another through either the presence or absence of a particular gene or fragment thereof.

By "presence" of a gene it is meant that the whole or part of the specified gene is detectable by methods well known to those skilled in the art.

For example, an insect cell-line can be differentiated from a mammalian cell-line through the detection of a gene only present in insect cells.

Accordingly, in one aspect there is a provided a method of cell-line identification comprising the step wherein the cell-line type is determined by the presence and/or absence of an attacin gene or fragment thereof.

Accordingly, in one aspect there is provided a method of cell-line identification comprising the step: PCR amplification of the pre-proattacin (PPATT) gene or fragment thereof. In a aspect, there is provided a method of cell-line identification comprising the step: PCR amplification of the pre-proattacin A (PPATTA) gene or fragment thereof. In one aspect there is provided a method wherein a fragment of the attacin gene is amplified by PCR. Accordingly, a method wherein a fragment of the PPATT gene is amplified using at least one of the primers comprising the sequences selected from SEQ ID NO: 5 and SEQ ID NO: 6 or functional derivative thereof.

However, it is clear to the person skilled in the art that the presence and or absence of a gene can be determined by a number of techniques including PCR amplification of the gene or part thereof. Other techniques by which the presence of genes may be assessed include but are not limited to DNA hybridisation, including *in situ* DNA hybridisation. As used herein, the terms "hybridisation" or "specific hybridisation" means that the primer or probe forms a duplex (double-stranded nucleotide sequence) with part of a target or with the entire region under the experimental conditions used, and that under those conditions the primer or probe does not form a duplex with other regions of the nucleotide sequence present in the sample to be analysed. It should be understood that the primers and probes of the present disclosure are designed for specific hybridisation to specific genes and may therefore fall entirely within said region or may to a large extent overlap with said region (i.e. form a duplex with nucleotides outside as well as within said region). Accordingly, there is provided a method in which the primers as herein described may be used during a PCR reaction or DNA hybridisation.

Polymerase Chain Reaction (PCR) amplification maybe performed from the genomic nucleic acid isolated and or purified from a cell-culture. Encompassed within the disclosure are PCR reactions carried out on a cell suspension, crude, isolated or purified genomic preparation, using techniques of genomic DNA preparation well known to those skilled in the art. The genomic preparations encompassed by the present disclosure may be isolated or substantially purified. By "isolated" or "substantially purified" is intended that the nucleic acid molecules, are substantially or essentially free from components normally found in association with the nucleic acid in its natural state. Such components include other cellular material and culture media for example.

Methods for designing PCR primers are generally known in the art and are disclosed in Sambrook and Russel, Molecular Cloning: A Laboratory Manual (Cold Harbour Laboratory Press). Known methods of PCR include, but are not limited to, methods using paired primers, nested primers, single specific primers, degenerate primers, gene-specific primers, vector-specific primers, partially mismatched primers, and the like.

With PCR, it is possible to amplify a single copy of a specific target sequence to a level detectable by several different methodologies (e.g., but not limited to, hybridization with a labelled probe; incorporation of biotinylated primers followed by avidin-enzyme conjugate detection; incorporation of 32P-labeled deoxynucleotide triphosphates, such as dCTP or dATP, into the amplified segment, incorporation of a fluorochrome such as etidium bromide, or other commercial compounds). In addition to genomic DNA, any nucleotide sequence can be amplified with the appropriate set of primer molecules. In particular, the amplified segments created by the PCR process itself are, themselves, efficient templates for subsequent PCR amplifications.

Amplification in PCR requires "PCR reagents" or "PCR materials," which herein are defined as all reagents necessary to carry out amplification except the polymerase, primers, and template. PCR reagents normally include nucleic acid precursors (dCTP, dTTP, etc.), and buffer.

The term "primer" is used herein to mean any single-stranded oligonucleotide sequence capable of being used as a primer in, for example, PCR technology. Thus, a 'primer' refers to a single-stranded oligonucleotide sequence that is capable of acting as a point of initiation for synthesis of a primer extension product that is complementary to the nucleic acid strand to be copied. The design (length and specific sequence) of the primer will depend on the nature of the DNA and/or RNA targets and on the conditions at which the primer is used (such as temperature and ionic strength).

The primers may consist of the nucleotide sequences shown in SEQ ID NO: 1 to 6, or may be 1, 2, 3, 4, 5, 6, 7, 8, 9 10, 15, 20, 25, 30, 35, 40, 45, 50, 75 or 100 or more bases which comprise or fall within the sequences of SEQ ID NO: 1 to 6, provided they are suitable for specifically binding DNA of target loci, under stringent conditions. When needed, slight modifications of the primers or probes in length or in sequence can be carried out to maintain the specificity and sensitivity required under the given circumstances and thus under 1, 2, 3, 4, 5, 6 or more nucleotides may be substituted. Probes and/ or primers listed herein may be extended by 1, 2, 3, 4 or 5 nucleotides, for example, in either direction.

For the avoidance of doubt one letter designation other than A, T, G and C mean the following:

| **Single letter code** |
|---|
| **B** = C or G or T |
| **D** = A or G or T |
| **H** = A or C or T |
| **K** = G or T |
| **M** = A or C |
| **N** = A or C or G or T |
| **R** = A or G |
| **S** = C or G |
| **V** = A or C or G |
| **W** = A or T |
| **Y** = C or T |

The term "functional derivative" is used herein to mean a primer that comprises a sequence that is at least 95% identical to a primer as defined herein over the length of the primer, suitably greater than 95% identical such as 96%, 97%, 98%, 99% and most preferably has 100% identity over its length. Functional derivatives may have 1, 2, 3, 4, 5, 6, 7, 8, 9 or 10 base deletions at the 5' end or 3' end or both.

As used herein, the term "stringent conditions" means any hybridisation conditions which allow the primers to bind to a specific nucleotide sequence, and not to any other loci on the cell genome. Such stringent conditions are known to those skilled in the art and can be found in *Current Protocols in Molecular Biology,* John Wiley & Sons, N.Y. (1989), 6.3.1-6.3.6. A non-limiting example of stringent hybridization conditions is hybridization at 6X sodium chloride/sodium citrate (SSC) at about 45° C, followed by one or more washes in 0.1XSSC, 0.2% SDS at about 68° C. An alternate example of stringent hybridization conditions are hybridization in 6XSSC at about 45° C, followed by one or more washes in 0.2 X SSC, 0.1% SDS at 50-65° C (*i.e.,* one or more washes at 50° C, 55° C, 60° C or 65° C).

The methods of the disclosure can be adapted according to the cell-lines that are required to differentiated or determined. For example, some cell-lines are capable of being differentiated by PCR amplification of an intron of calmodulin alone. However, some cell-lines may result in an amplicon that is the same size or as another cell-line and thus further steps are required for cell-line identification.

Accordingly, in one aspect there is provided a cell-line identification method comprising the steps: PCR amplification of the calmodulin gene or fragment thereof.

In a further aspect there is provided a cell-line identification method comprising the steps: PCR amplification of the attacin gene or fragment thereof.

In a further aspect there is provided a cell-line identification method comprising the steps: PCR amplification of the Axl receptor tyrosine kinase gene or fragment thereof.

In a further aspect there is provided a cell-line identification method comprising the following steps: (a) PCR amplification of the calmodulin gene or fragment thereof; and (b) PCR amplification of an attacin gene or fragment thereof.

In a further aspect there is provided a cell-line identification method comprising the following steps: (a) PCR amplification of the calmodulin gene or fragment thereof; and (b) PCR amplification of the Axl receptor tyrosine kinase gene or fragment thereof.

In a further embodiment there is provided a cell-line identification method comprising the following steps: (a) PCR amplification of an attacin gene or fragment thereof; and (b) PCR amplification of an Axl receptor tyrosine kinase gene or fragment thereof.

In one aspect there is provided a cell-line identification method comprising the following steps: (a) PCR amplification of the calmodulin gene or fragment thereof; (b) PCR amplification of an attacin gene or fragment thereof; and (c) PCR amplification of the Axl receptor tyrosine kinase gene or fragment thereof.

In a particular aspect, the PCR reactions may be performed in a single PCR reaction. Accordingly, one aspect provides a method in which the PCR amplification reactions are multiplexed. As used herein, the term "multiplexed" means any number of PCR reaction are performed in a single reaction tube *i.e*. the contents of a single reaction tube comprises more than 1 set of primers. Primers of the disclosure are designed so that a single PCR reaction can be performed using specified reaction conditions allowing amplifications of each gene or fragment thereof used in the cell-identification method (Calmodulin, Axl receptor tyrosine kinase and attacin) without prejudice to the amplification of another gene. Multiplexing allows a faster and easier method of molecular identification of cell-line as described herein.

The methods of the disclosure are suitable for the identification of a variety of cell-lines comprising a calmodulin, Axl receptor tyrosine kinase or attacin gene. In one aspect there is provided a method for identification cell-lines used in the pharmaceutical industry, particularly I the production of vaccines. In one aspect there is provided a method for identification cell-lines MRC-5, Vero, Hi-5, CHO, FRHL2, CEF and MDCK. These cell-lines are well known to those skilled in the art, however to summarise:

| **Cell line** | **Organism** |
|---|---|
| CHO | *Cricetulus griseus* |
| | Chinese Hamster |
| | |
| MRC-5 | *Homo sapiens* |
| | Human |
| | |
| MDCK | *Canis familiaris* |
| | Dog |
| | |
| Vero | *Cercopithecus aethiops* |
| | African Green Monkey |
| FRHL2 | *Macaca mulatta* |
| | Rhesus Monkey |
| | |
| Chicken | *Gallus gallus* |
| | |
| Hi-5 | *Trichoplusia ni* |
| | Lepidoptera Insect |

In a further aspect, there is provided a kit for cell-line identification comprising primers complementary to any one of: the calmodulin gene; an Axl receptor tyrosine kinase gene; or an attacin gene; or any combination thereof. In a further aspect the kit comprises at least one primer comprising or consisting of the following sequences or functional derivative thereof: SEQ ID NO: 1, SEQ ID NO: 2, SEQ ID NO: 3, SEQ ID NO: 4, SEQ ID NO: 5 or SEQ ID NO: 6 or functional derivative thereof.

In one aspect there is provided the use of a calmodulin gene, a tyrosine kinase gene and/or the attacin gene or any combination thereof in cell-line identification.

### EXAMPLES

### 1.1 Extraction of genomic DNA and PCR amplification

Genomic DNA was extracted from CHO, MRC-5, MDCK, VERO, FRHL2, Hi-5 and *Gallus* [CFO] cells using the High Pure PCR template Preparation Kit (Roche). PCR was then performed on the under the following reaction conditions using the primers Calm ex 3F (SEQ ID NO:1 Calm ex 4R (SEQ ID NO:2), Axl ex 18F (SEQ ID NO:3), Axl 19R (SEQ ID NO:4), ppatta F (SEQ ID NO:5) and ppatta R (SEQ ID NO:6):

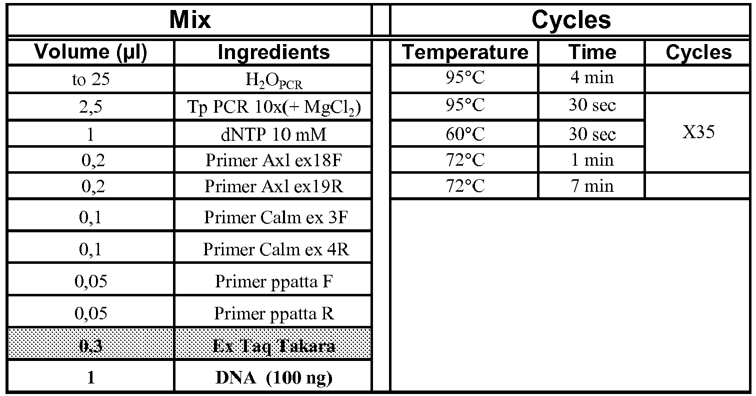

### 2.1 Sizing of the PCR products by capillary electrophoresis

The following example demonstrates that the difference in the size of the intron between exons 3 and 4 of calmodulin and 18 and 19 of Axl, can unequivocally identify the 7 different cell lines form which genomic DNA was extracted (see 1.1).

In order to allow the detection of the DNA products after capillary electrophoresis, the reverse primers used during the PCR amplification were labelled with distinct fluorescent dyes; the reverse primer for PPTTA was labelled with NED, the reverse primer for calmodulin was labelled with Hex and the Axl reverse primer was labelled with 6-FAM. The resulting labelled DNA fragments were run in parallel with a size marker, allowing the analysis of the PCR product with a size resolution around 1-bp for fragments of less than 500-bp.

Genomic DNA was amplified using fluorescent reverse primers at a hybridization temperature of 60°C (according to the PCR conditions described above). The capillary electrophoresis was performed with the Genetic Analyzer instrument (ABl3730) using 50 cm capillaries (ROX-1200 ladder, ABI).

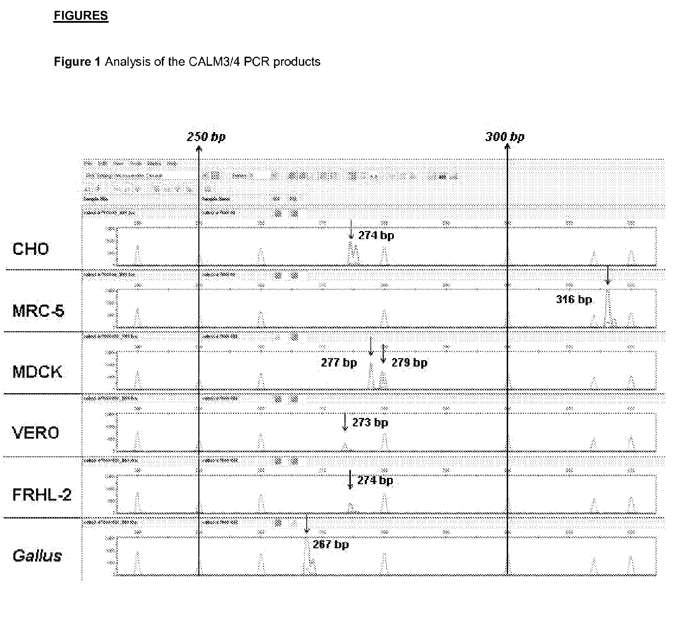

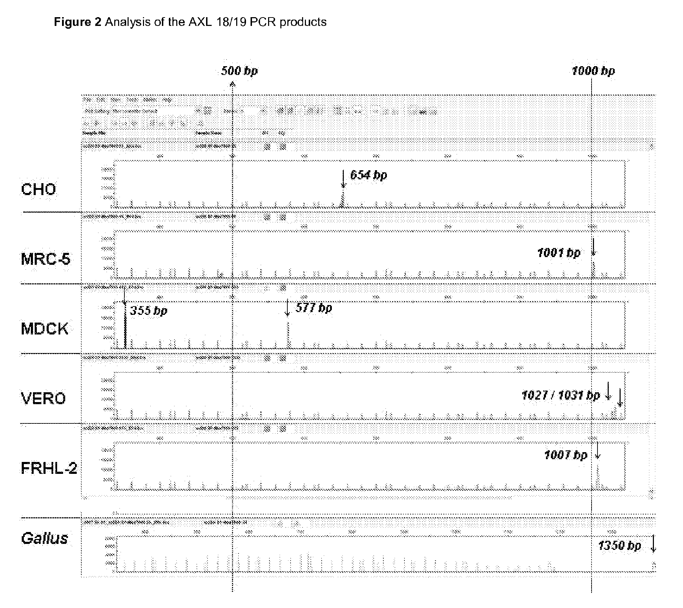

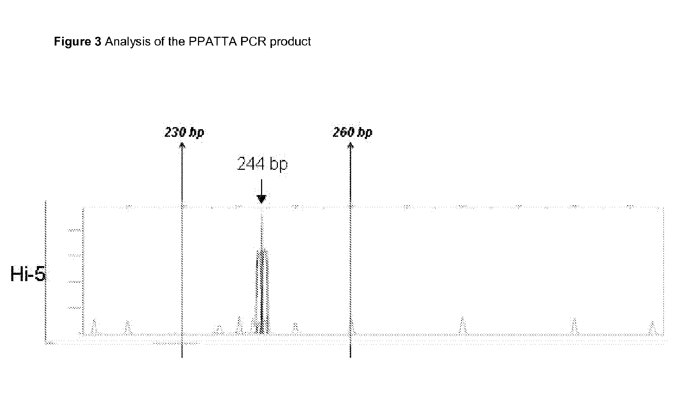

The sizes of the amplified products were compared to the expected sizes in **Table 1**. The capillary electrophoresis profiles are presented in **Figures 1 to 3**.

**Table 1: Comparison of the PCR product sizes determined by capillary electrophoresis (observed from the cells) with the sizes deduced from the published sequences (expected from the organism).**

| **Cells** | **PPATTA** | | **CALM3/4** | | **AXL18/19** | |
|---|---|---|---|---|---|---|
| **Size (bp)** | **Observed Cells** | **Expected Organism** | **Observed Cells** | **Expected Organism** | **Observed Cells** | **Expected Organism** |
| **MRC-5** | NA | NA | **316** | **320** | **1001** | **1010** |
| ***VERO*** | NA | NA | **273** | **ND** | **1027+1031** | **ND** |
| ***CHO*** | NA | NA | **274** | **ND** | **654** | **ND** |
| **FRHL2** | NA | NA | **274** | **278** | **1007** | **1021** |
| **MDCK** | NA | NA | **277+279** | **283** | **355+577** | **583** |
| **Chicken** | NA | NA | **267** | **270** | **1350** | **1489** |
| **Hi-5** | **244** | **235** | NA | NA | NA | NA |

| | | | | | | |
|---|---|---|---|---|---|---|
| NA: Not applicable **ND:** Not described | | | | | | |

### CALM3/4 (amplification of the calmodulin gene between exons 3 and 4) PCR products

The size of the PCR products amplified from MRC-5, FRHL-2, MDCK and Chicken cells were very close to the predicted sizes. Two peaks were observed from MDCK cells (277 and 279-bp).

A 274bp fragment was amplified from CHO and FHRL-2 cells, close to the 273-bp fragment amplified from VERO cells. Other fragments were different by at least 3 base pairs. The sizes of the VERO and CHO fragments were not known before performing the experiment.

### AXL18/19 (amplification of the calmodulin gene between exons 18 and 19) PCR products

AXL 18/19 PCR allowed the discrimination of all 6 vertebrate DNAs. The size of the products amplified from VERO (1027 and 1031-bp) and CHO (654-bp) cells were clearly different.

### PPATTA (attacin) PCR products

The size of the DNA fragment amplified from *T. ni* DNA using the PPATTA primers was estimated at 244-bp. This size was matching with the expected 235-bp.

### Conclusions

The combined analysis of CALM3/4 and AXL18/19 PCR products yielded size patterns allowing the unequivocal identification of the 6 vertebrate cell lines.

### 3.1 Ability to detect a minor species i.e. contamination

The objective of the following example was to evaluate the capacity of the PCR assay above to detect a mix of 2 cell different types wherein the major species represents 90%, and the minor species represents 10% of the mix.

The 90/10% mixes of different cell types were performed at the level of the genomic DNA considering the number of genome copies (see **Table 2**)

The number of genome copies was calculated from the amount of DNA (measured by the Threshold system) and from the genome sizes (http://www.genomesize.com)

**Table 2: Determination of the number of genome copies**

| **Cell line** | **Haploid genome size (qg)** | **DNA concentration (ng/µl)** | **Volume in 100 µl to obtain 9000 copies / µl** | **Volume in 100 µl to obtain 1000 copies / µl** |
|---|---|---|---|---|
| **MRC-5** | 3,5 | 83 | 37,8 | 4,2 |
| **Vero** | 3,5 | 58 | 54 | 6 |
| **Hi-5** | 0,5 | 96 | 5 | 0,5 |
| **CHO** | 3,5 | 69 | 45 | 5 |
| **FRHL2** | 3,5 | 126 | 25,2 | 2,8 |
| **MDCK** | 3,2 | 34 | 85,5 | 9,5 |
| **Chicken** | 1,25 | 38 | 29,7 | 3,3 |

Approximately 27,000 copies of the major species and 3,000 copies of the minor species were mixed and amplified under the PCR conditions described above (see 1.1)

### Results

The detection results from the different mixes are summarized in the **Table 3**.

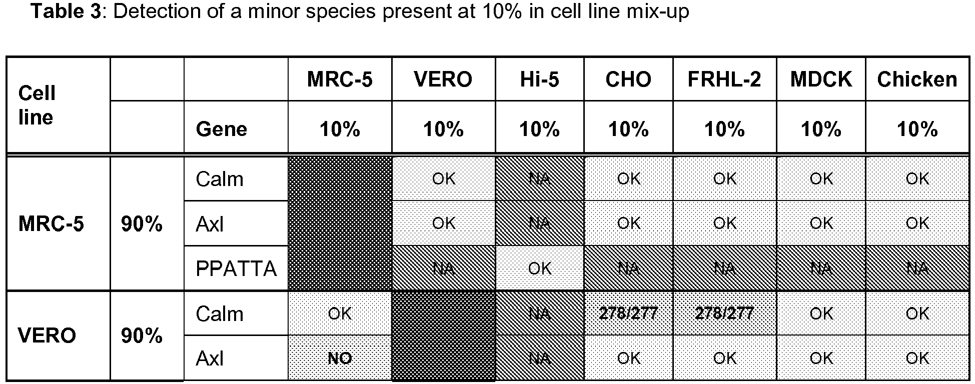

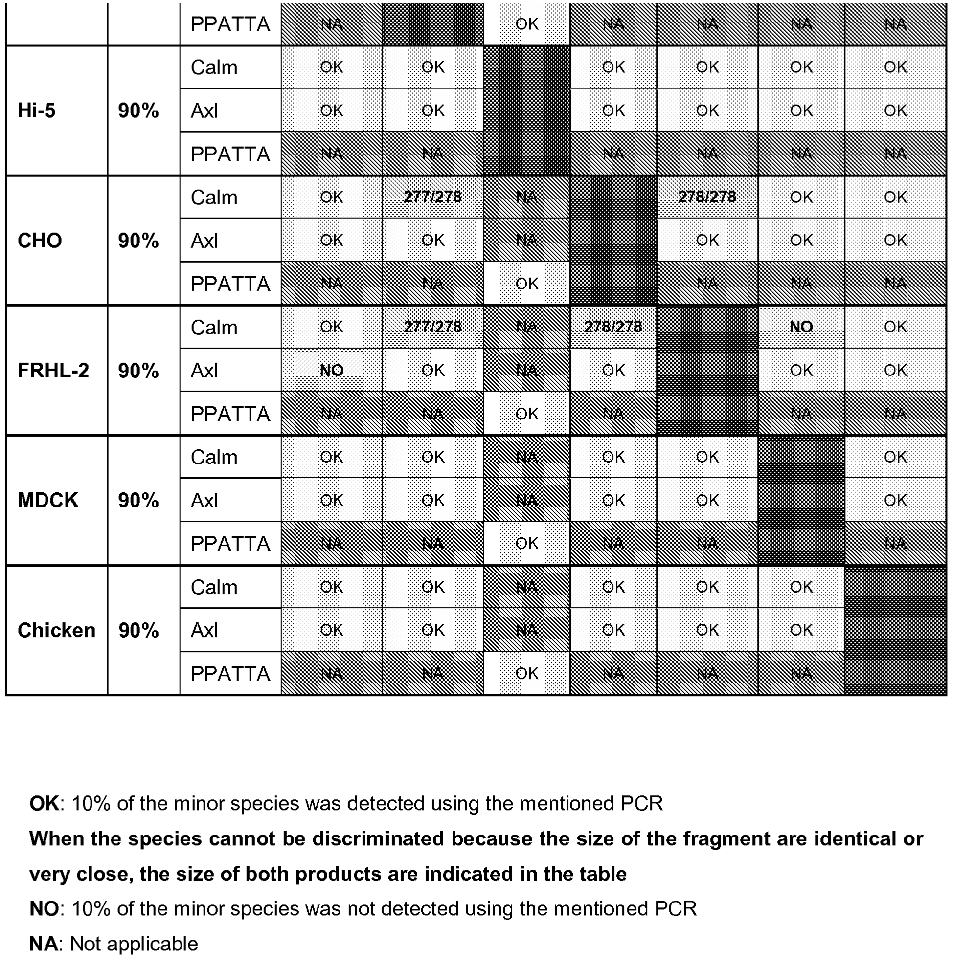

For some mixes, a single PCR amplification did not allow to detect the minor species:
- CHO/VERO/FRHL2 mixes could not be detected using the CALM3/4 PCR because the products displayed identical or similar sizes
- 10% MDCK in FRHL-2 could not be detected with the CALM3/4 assay
- 10% MRC5 in FRHL-2 and in VERO could not be detected with the AXL18/19 assay

It can thus be seen that the combined analysis of CALM3/4, AXL18/19 and PPATTA PCR products allowed the detection of a 10% DNA contamination between all different 7 cell lines, and that in some instances only 1 or 2 genes analyses were required to detect the contamination.

## Claims

1. A method of cell-line identification comprising the following steps:
(a) analysis of the calmodulin gene, wherein a fragment of the calmodulin gene is amplified by PCR and wherein the amplified fragment comprises the sequence between exon 3 and exon 4 of the calmodulin gene; and
(b) analysis of the Axl receptor tyrosine kinase gene, wherein a fragment of the Axl receptor tyrosine kinase gene is amplified by PCR, and wherein the amplified fragment comprises the sequence between exon 18 and exon 19 of the Axl receptor tyrosine kinase gene;
and wherein the cell-line is identified by the size of an intron in said genes.

2. The method of claim 1 wherein the calmodulin gene is a nucleic acid sequence that encodes a polypeptide of SEQ ID NO: 7 or encodes an amino acid sequence having 70%, 75%, 80%, 85%, 90%, 95%, 96%, 97%, 98% or 99% sequence identity to SEQ ID NO:7.

3. The method of claim 1 or 2 wherein the Axl receptor tyrosine kinase gene is nucleic acid sequence that encodes a polypeptide of SEQ ID NO: 8 or polypeptides with amino acid sequences that have 70%, 75%, 80%, 85%, 90%, 95%, 96%, 97%, 98% or 99% sequence identity to SEQ ID NO: 8.

## Patentansprüche

1. Verfahren zur Zelllinienidentifikation, umfassend die folgenden Schritte:
(a) Analyse des Calmodulin-Gens, worin ein Fragment des Calmodulin-Gens durch PCR amplifiziert wird und worin das amplifizierte Fragment die Sequenz zwischen Exon 3 und Exon 4 des Calmodulin-Gens umfasst; und
(b) Analyse der AxI-Rezeptor-Tyrosinkinase-Gens, worin ein Fragment des AxI-Rezeptor-Tyrosinkinase-Gens durch PCR amplifiziert wird, und worin das amplifizierte Fragment die Sequenz zwischen Exon 18 und Exon 19 des AxI-Rezeptor-Tyrosinkinase-Gens umfasst;
und worin die Zelllinie durch die Größe eines Introns in in den genannten Genen identifiziert wird.

2. Verfahren nach Anspruch 1, worin das Calmodulin-Gen eine Nukleinsäuresequenz ist, die ein Polypeptid SEQ ID NO:7 oder eine Aminosäuresequenz mit 70%, 75%, 80%, 85%, 90%, 95%, 96%, 97%, 98% oder 99% Sequenzidentität zur SEQ ID NO:7 kodiert.

3. Verfahren nach Anspruch 1 oder 2, worin das AxI-Rezeptor-Tyrosinkinase-Gen eine Nukleinsäuresequenz ist, die ein Polypeptid SEQ ID NO:8 oder Polypeptide mit Aminosäuresequenzen kodiert, die 70%, 75%, 80%, 85%, 90%, 95%, 96%, 97%, 98% oder 99% Sequenzidentität zur SEQ ID NO:8 besitzen.

## Revendications

1. Procédé d'identification de lignée cellulaire comprenant les étapes suivantes :
(a) l'analyse du gène de la calmoduline, où un fragment du gène de la calmoduline est amplifié par PCR et où le fragment amplifié comprend la séquence entre l'exon 3 et l'exon 4 du gène de la calmoduline ; et
(b) l'analyse du gène du récepteur tyrosine kinase Axl, où un fragment du gène du récepteur tyrosine kinase Axl est amplifié par PCR, et où le fragment amplifié comprend la séquence entre l'exon 18 et l'exon 19 du gène du récepteur tyrosine kinase Axl ;
et dans lequel la lignée cellulaire est identifiée par la taille d'un intron dans lesdits gènes.

2. Procédé selon la revendication 1, dans lequel le gène de la calmoduline est une séquence d'acide nucléique qui code pour un polypeptide de SEQ ID NO : 7 ou qui code pour une séquence d'acides aminés présentant 70 %, 75 %, 80 %, 85 %, 90 %, 95 %, 96 %, 97 %, 98 % ou 99 % d'identité de séquence avec SEQ ID NO : 7.

3. Procédé selon la revendication 1 ou 2, dans lequel le gène du récepteur tyrosine kinase Axl est une séquence d'acide nucléique qui code pour un polypeptide de SEQ ID NO : 8 ou pour des polypeptides avec des séquences d'acides aminés qui présentent 70 %, 75 %, 80 %, 85 %, 90 %, 95 %, 96 %, 97 %, 98 % ou 99 % d'identité de séquence avec SEQ ID NO : 8.


### SEQUENCE LISTING

<110> Cassart, Jean-Pol Lienard, Patricia
<120> Novel Method
<130> VB63206P
<160> 9
<170> FastSEQ for Windows Version 4.0
<210> 1
   <211> 27
   <212> DNA
   <213> Artificial Sequence
<220>
   <223> oligonucleotide
<400> 1
   caaagaagcy ttttcactat ttgacaa 27
<210> 2
   <211> 26
   <212> DNA
   <213> Artificial Sequence
<220>
   <223> oligonucleotide
<400> 2
   tcatttttct tgccatcatt gtcaga 26
<210> 3
   <211> 20
   <212> DNA
   <213> Artificial Sequence
<220>
   <223> oligonucleotide
<400> 3
   ggrgactact accgycaggg 20
<210> 4
   <211> 22
   <212> DNA
   <213> Artificial Sequence
<220>
   <223> oligonucleotide
<400> 4
   caatctccca catbgtcacc cc 22
<210> 5
   <211> 25
   <212> DNA
   <213> Artificial Sequence
<220>
   <223> oligonucleotide
<400> 5
   ctattgtcga ccatacctct accgt 25
<210> 6
   <211> 20
   <212> DNA
   <213> Artificial Sequence
<220>
   <223> oligonucleotide
<400> 6
   cgtggccgtt gctggataag 20
<210> 7
   <211> 149
   <212> PRT
   <213> H. sapien
<400> 7
<210> 8
   <211> 894
   <212> PRT
   <213> H. sapien
<400> 8
   Met Ala Trp Arg Cys Pro Arg Met Gly Arg Val Pro Leu Ala Trp Cys
    1               5                  10                  15
   Leu Ala Leu Cys Gly Trp Ala Cys Met Ala Pro Arg Gly Thr Gln Ala
               20                  25                  30
   Glu Glu Ser Pro Phe Val Gly Asn Pro Gly Asn Ile Thr Gly Ala Arg
           35                  40                  45
   Gly Leu Thr Gly Thr Leu Arg Cys Gln Leu Gln Val Gln Gly Glu Pro
       50                  55                  60
   Pro Glu Val His Trp Leu Arg Asp Gly Gln Ile Leu Glu Leu Ala Asp
   65                  70                  75                  80
   Ser Thr Gln Thr Gln Val Pro Leu Gly Glu Asp Glu Gln Asp Asp Trp
                   85                  90                  95
   Ile Val Val Ser Gln Leu Arg Ile Thr Ser Leu Gln Leu Ser Asp Thr
               100                 105                 110
   Gly Gln Tyr Gln Cys Leu Val Phe Leu Gly His Gln Thr Phe Val Ser
           115                 120                 125
   Gln Pro Gly Tyr Val Gly Leu Glu Gly Leu Pro Tyr Phe Leu Glu Glu
       130                 135                 140
   Pro Glu Asp Arg Thr Val Ala Ala Asn Thr Pro Phe Asn Leu Ser Cys
   145                 150                 155                 160
   Gln Ala Gln Gly Pro Pro Glu Pro Val Asp Leu Leu Trp Leu Gln Asp
                   165                 170                 175
   Ala Val Pro Leu Ala Thr Ala Pro Gly His Gly Pro Gln Arg Ser Leu
               180                 185                 190
   His Val Pro Gly Leu Asn Lys Thr Ser Ser Phe Ser Cys Glu Ala His
           195                 200                 205
   Asn Ala Lys Gly Val Thr Thr Ser Arg Thr Ala Thr Ile Thr Val Leu
       210                 215                 220
   Pro Gln Gln Pro Arg Asn Leu His Leu Val Ser Arg Gln Pro Thr Glu
   225                 230                 235                 240
   Leu Glu Val Ala Trp Thr Pro Gly Leu Ser Gly Ile Tyr Pro Leu Thr
                   245                 250                 255
   His Cys Thr Leu Gln Ala Val Leu Ser Asp Asp Gly Met Gly Ile Gln
               260                 265                 270
   Ala Gly Glu Pro Asp Pro Pro Glu Glu Pro Leu Thr Ser Gln Ala Ser
           275                 280                 285
   Val Pro Pro His Gln Leu Arg Leu Gly Ser Leu His Pro His Thr Pro
       290                 295                 300
   Tyr His Ile Arg Val Ala Cys Thr Ser Ser Gln Gly Pro Ser Ser Trp
   305                 310                 315                 320
   Thr His Trp Leu Pro Val Glu Thr Pro Glu Gly Val Pro Leu Gly Pro
                   325                 330                 335
   Pro Glu Asn Ile Ser Ala Thr Arg Asn Gly Ser Gln Ala Phe Val His
               340                 345                 350
   Trp Gln Glu Pro Arg Ala Pro Leu Gln Gly Thr Leu Leu Gly Tyr Arg
           355                 360                 365
   Leu Ala Tyr Gln Gly Gln Asp Thr Pro Glu Val Leu Met Asp Ile Gly
       370                 375                 380
   Leu Arg Gln Glu Val Thr Leu Glu Leu Gln Gly Asp Gly Ser Val Ser
   385                 390                 395                 400
   Asn Leu Thr Val Cys Val Ala Ala Tyr Thr Ala Ala Gly Asp Gly Pro
                   405                 410                 415
   Trp Ser Leu Pro Val Pro Leu Glu Ala Trp Arg Pro Gly Gln Ala Gln
               420                 425                 430
   Pro Val His Gln Leu Val Lys Glu Pro Ser Thr Pro Ala Phe Ser Trp
           435                 440                 445
   Pro Trp Trp Tyr Val Leu Leu Gly Ala Val Val Ala Ala Ala Cys Val
       450                 455                 460
   Leu Ile Leu Ala Leu Phe Leu Val His Arg Arg Lys Lys Glu Thr Arg
   465                 470                 475                 480
   Tyr Gly Glu Val Phe Glu Pro Thr Val Glu Arg Gly Glu Leu Val Val
                   485                 490                 495
   Arg Tyr Arg Val Arg Lys Ser Tyr Ser Arg Arg Thr Thr Glu Ala Thr
               500                 505                 510
   Leu Asn Ser Leu Gly Ile Ser Glu Glu Leu Lys Glu Lys Leu Arg Asp
           515                 520                 525
   Val Met Val Asp Arg His Lys Val Ala Leu Gly Lys Thr Leu Gly Glu
       530                 535                 540
   Gly Glu Phe Gly Ala Val Met Glu Gly Gln Leu Asn Gln Asp Asp Ser
   545                 550                 555                 560
   Ile Leu Lys Val Ala Val Lys Thr Met Lys Ile Ala Ile Cys Thr Arg
                   565                 570                 575
   Ser Glu Leu Glu Asp Phe Leu Ser Glu Ala Val Cys Met Lys Glu Phe
               580                 585                 590
   Asp His Pro Asn Val Met Arg Leu Ile Gly Val Cys Phe Gln Gly Ser
           595                 600                 605
   Glu Arg Glu Ser Phe Pro Ala Pro Val Val Ile Leu Pro Phe Met Lys
       610                 615                 620
   His Gly Asp Leu His Ser Phe Leu Leu Tyr Ser Arg Leu Gly Asp Gln
   625                 630                 635                 640
   Pro Val Tyr Leu Pro Thr Gln Met Leu Val Lys Phe Met Ala Asp Ile
                   645                 650                 655
   Ala Ser Gly Met Glu Tyr Leu Ser Thr Lys Arg Phe Ile His Arg Asp
               660                 665                 670
   Leu Ala Ala Arg Asn Cys Met Leu Asn Glu Asn Met Ser Val Cys Val
           675                 680                 685
   Ala Asp Phe Gly Leu Ser Lys Lys Ile Tyr Asn Gly Asp Tyr Tyr Arg
       690                 695                 700
   Gln Gly Arg Ile Ala Lys Met Pro Val Lys Trp Ile Ala Ile Glu Ser
   705                 710                 715                 720
   Leu Ala Asp Arg Val Tyr Thr Ser Lys Ser Asp Val Trp Ser Phe Gly
                   725                 730                 735
   Val Thr Met Trp Glu Ile Ala Thr Arg Gly Gln Thr Pro Tyr Pro Gly
               740                 745                 750
   Val Glu Asn Ser Glu Ile Tyr Asp Tyr Leu Arg Gln Gly Asn Arg Leu
           755                 760                 765
   Lys Gln Pro Ala Asp Cys Leu Asp Gly Leu Tyr Ala Leu Met Ser Arg
       770                 775                 780
   Cys Trp Glu Leu Asn Pro Gln Asp Arg Pro Ser Phe Thr Glu Leu Arg
   785                 790                 795                 800
   Glu Asp Leu Glu Asn Thr Leu Lys Ala Leu Pro Pro Ala Gln Glu Pro
                   805                 810                 815
   Asp Glu Ile Leu Tyr Val Asn Met Asp Glu Gly Gly Gly Tyr Pro Glu
               820                 825                 830
   Pro Pro Gly Ala Ala Gly Gly Ala Asp Pro Pro Thr Gln Pro Asp Pro
           835                 840                 845
   Lys Asp Ser Cys Ser Cys Leu Thr Ala Ala Glu Val His Pro Ala Gly
       850                 855                 860
   Arg Tyr Val Leu Cys Pro Ser Thr Thr Pro Ser Pro Ala Gln Pro Ala
   865                 870                 875                 880
   Asp Arg Gly Ser Pro Ala Ala Pro Gly Gln Glu Asp Gly Ala
                   885                 890
<210> 9
   <211> 192
   <212> PRT
   <213> Trichoplusia
<400> 9
   Gln Ala Gln Gly Ser Val Thr Leu Asn Ser Asp Gly Ser Met Gly Leu
   1               5                   10                  15
   Gly Ala Lys Val Pro Ile Val Gly Asn Glu Lys Asn Val Leu Ser Ala
               20                  25                  30
   Leu Gly Ser Val Asp Leu Asn Asp Gln Leu Lys Pro Ala Ser Arg Gly
           35                  40                  45
   Met Gly Leu Ala Leu Asp Asn Val Asn Gly His Gly Leu Ser Val Met
       50                  55                  60
   Lys Glu Thr Val Pro Gly Phe Gly Asp Arg Leu Thr Gly Ala Gly Arg
   65                  70                  75                  80
   Val Asn Val Phe His Asn Asp Asn His Asp Ile Ser Ala Lys Ala Phe
                   85                  90                  95
   Val Thr Lys Asn Met Pro Asp Phe Pro Asn Val Pro Asn Phe Asn Thr
               100                 105                 110
   Val Gly Gly Gly Val Asp Tyr Met Tyr Lys Asn Lys Val Gly Ala Ser
           115                 120                 125
   Leu Gly Met Ala Asn Thr Pro Phe Leu Asp Arg Lys Asp Tyr Ser Ala
       130                 135                 140
   Met Gly Asn Leu Asn Val Phe Arg Ser Pro Thr Thr Ser Val Asp Phe
   145                 150                 155                 160
   Asn Ala Gly Phe Lys Lys Phe Asp Thr Pro Val Phe Lys Ser Asn Trp
                   165                 170                 175
   Glu Pro Asn Phe Gly Leu Thr Phe Ser Arg Ser Phe Gly Asn Lys Trp
               180                 185                 190